Europäisches Patentamt

European Patent Office

Office européen des brevets

(19)

(11) **EP 1 077 681 B1**

## (12) EUROPÄISCHE PATENTSCHRIFT

(45) Veröffentlichungstag und Bekanntmachung des Hinweises auf die Patenterteilung:
**09.10.2002 Patentblatt 2002/41**

(21) Anmeldenummer: **99922182.3**

(22) Anmeldetag: **05.05.1999**

(51) Int Cl.$^7$: **A61K 9/20**, A61K 31/195

(86) Internationale Anmeldenummer:
**PCT/EP99/03087**

(87) Internationale Veröffentlichungsnummer:
**WO 99/059551 (25.11.1999 Gazette 1999/47)**

(54) **LEVOTHYROXIN NATRIUM ENTHALTENDE PHARMAZEUTISCHE ZUBEREITUNG**

PHARMACEUTICAL PREPARATION CONTAINING LEVOTHYROXINE SODIUM

PREPARATION PHARMACEUTIQUE CONTENANT DE LA LEVOTHYROXINE SODIQUE

(84) Benannte Vertragsstaaten:
**AT BE CH DE DK ES FI FR GB GR IE IT LI LU NL PT SE**
Benannte Erstreckungsstaaten:
**LT LV RO SI**

(30) Priorität: **15.05.1998 DE 19821625**

(43) Veröffentlichungstag der Anmeldung:
**28.02.2001 Patentblatt 2001/09**

(73) Patentinhaber: **MERCK PATENT GmbH**
**64293 Darmstadt (DE)**

(72) Erfinder:
• **SCHREDER, Sven**
**D-69115 Heidelberg (DE)**
• **NISCHWITZ, Marion**
**D-64291 Darmstadt (DE)**

(56) Entgegenhaltungen:
**DE-A- 19 541 128          GB-A- 1 180 574**
**US-A- 5 225 204**

• **DATABASE WPI Section Ch, Week 9749 Derwent Publications Ltd., London, GB; Class A96, AN 97-527238 XP002113271 & CN 1 126 589 A (CHENGDU INST ORGANIC CHEM CHINESE ACAD), 17. Juli 1996 (1996-07-17)**

**Beschreibung**

Gegenstand der Erfindung ist eine neue stabile pharmazeutische Zubereitung enthaltend Levothyroxin Natrium, Gelatine und Füllstoffe und die frei von organischen Lösungsmittelrückständen ist. Es handelt sich um eine feste Zubereitung in Form von Tabletten.

Diese neue Zubereitung hat eine verbesserte Stabilität und kann als Thyroidhormon-Präparat verwendet werden.

Femer hat diese neue Zubereitung eine verbesserte Wirkstoff-Freisetzung in vitro.

Der Erfindung lag die Aufgabe zugrunde, neue Arzneimittel in Form von pharmazeutischen Zubereitungen zur Verfügung zu stellen, die bessere Eigenschaften besitzen als bekannte, für die gleichen Zwecke verwendbare Arzneimittel.

Das Department Of Health And Human Services, Food and Drug Administration, veröffentlichte im Federal Register Vol. 62, No. 15, August 14, 1997; Seite 43535, daß die im US-Markt verfügbaren Produkte, die Levothyroxin Natrium enthalten und oral verabreicht werden, Stabilitätsprobleme aufweisen und daher bis zu 20% überdosiert vorliegen und daß Hersteller entsprechende neue Anwendungsformen entwickeln müssen. Weiterhin sind die Anforderungen an die in-vitro-Freisetzung für Levothyroxin-Na Tabletten erhöht worden. Der Monographie-Entwurf des Pharmacopeial Forum (Pharm. Preview, **1995**, *21*, 1459-1461) sieht vor, neben dem gültigen Test 1 (Phosphatpuffer pH 7,4, in 80 Minuten > 55 %) den Test 2 (Wasser in 45 Minuten > 70%) zuzulassen.

Diese Aufgabe wurde durch das Auffinden der neuen Zubereitung gelöst.

Thyroxinhaltige Präparationen mit anderen Zusatzstoffen wie Glycin, einem Kohlenhydrat und einem anorganischen Salz sind aus der WO 97 17 951 bekannt.

Ein anderes mit Thiosulfat stabilisiertes Thyroxinpräparat ist in der DE 195 41 128 beschrieben.

Eine Kombinationspräparat enthaltend Levothyroxin Natrium und Kaliumiodid kennt man aus US 5,635,209.

Eine andere thyroxinhaltige Formulierung, die Thyroxin/Cyclodextrin-Komplexe enthält, ist in der WO 97/19703 beschrieben.

US 5,225,204 beschreibt einen stabilisierten Komplex aus Levothyroxin Natrium und wasserlöslichem Polyvinylpyrollidon adsorbiert an ein Cellulosederivat, der zu Tabletten verpresst oder in Kapseln abgefüllt werden kann.

Die erfindungsgemäße pharmazeutische Formulierung kann neben Levothyroxin Natrium auch Liothyronin Natrium enthalten.

Gegenstand der Erfindung ist vorzugsweise eine pharmazeutische Zubereitung wie beschrieben, dadurch gekennzeichnet, daß sie 5 bis 400 µg, vorzugsweise 10 bis 300 µg, insbesondere 25 bis 300 µg an Levothyroxin Natrium enthält.

Gegenstand der Erfindung ist ferner vorzugsweise eine pharmazeutische Zubereitung wie beschrieben, dadurch gekennzeichnet, daß sie Levothyroxin Natrium mikronisiert mit einer Korngröße zwischen 5 µm und 25 µm enthält.

Gegenstand der Erfindung ist weiterhin vorzugsweise eine pharmazeutische Zubereitung wie beschrieben, dadurch gekennzeichnet, daß sie Füllstoffe, ausgewählt aus der Gruppe Lactose und/oder Maisstärke und/oder mikrokristalline Cellulose enthält.

Besonders bevorzugte Ausführungsformen enthalten 25, 50, 75, 100, 125, 150, 175 oder 200 µg an Levothyroxin Natrium.

Der/die Wirkstoff/e ist/sind gegenüber Licht, Wärme und Sauerstoff empfindlich. Aufgrund dieser bekannten Instabilität wird in den Formulierungen der Wirkstoff zu 5 % überdosiert.

Die erfindungsgemäße Zubereitung weist eine überraschende Stabilität auf, wenn als Bindemittel Gelatine verwendet wird.

Wird diese durch ein anderes gängiges Bindemittel wie Methocel ersetzt, so stellt man schon bei Beginn der Stabilitätsuntersuchungen einen Rückgang des Wirkstoffgehalts fest, ferner ist die Summe der Nebenprodukte erhöht.

Wird z.B. bei einer 100 µg Charge, worin Gelatine durch Methocel ausgetauscht wurde, der Anfangswert an Wirkstoff bestimmt, so findet man anstelle der zu erwartenden 105 % nur 100,48 %.

Stabilitätsuntersuchungen zeigen, daß die erfindungsgemäßen Tabletten, die Levothyroxin Natrium enthalten, mindestens 2 Jahre stabil sind, wenn sie bei Temperaturen unter 30° C gelagert werden.

Ferner wird überraschenderweise die Freisetzung des Wirkstoffs Levothyroxin Natrium begünstigt, wenn der Wirkstoff in mikronisierter Form eingesetzt wird. Levothyroxin Natrium ist üblicherweise sowohl in Wasser und auch in Ethanol sehr schwer löslich. Mit einer Partikelgröße zwischen 5 µm und 25 µm (zu 95%) erfolgt jedoch eine Freisetzung des Wirkstoffs in Test 1 zu > 90% (Phosphatpuffer), in Test 2 zu > 80% (Wasser).

Überraschenderweise kann die erfindungsgemäße Zusammensetzung auch ohne die Verwendung organischer Lösungsmittel hergestellt werden. Wird das im erfindungsgemäßen Verfahren benutzte Wasser durch ein organisches Lösungsmittel wie z.B. Methanol ersetzt, so zeigt sich außerdem bei Testchargen nach 1 Jahr bei 25° C Lagertemperatur und 60 % rel. Feuchte ein Rückgang des Gehaltes an Levothyroxin Natrium um 10 %.

Als Füllstoffe für die erfindungsgemäße pharmazeutische Zubereitung eignen sich vorzugsweise Lactose, Maisstärke und/oder mikrokristalline Cellulose, sowohl als Einzelfüllstoffe als auch in Kombinationen untereinander. Besonders bevorzugte pharmazeutische Zubereitungen, wie beschrieben, enthalten Maisstärke und Lactose.

Gegenstand der Erfindung ist auch ein Verfahren zur Herstellung einer pharmazeutischen Zubereitung enthaltend Levothyroxin Natrium und gegebenenfalls Liothyronin Natrium, dadurch gekennzeichnet, daß man in einer Wirbelschichtgranulation Levothyroxin Natrium und gegebenenfalls Liothyronin Natrium, das/die in suspendierter Form in wäßriger Gelatinelösung vorliegt/vorliegen, auf den/die Füllstoff(e) aufsprüht, anschließend ein Spreng- und Schmiermittel zumischt und die Mischung zu Tabletten verpresst.

Gegenstand der Erfindung ist weiter ein Verfahren wie beschrieben, dadurch gekennzeichnet, daß als Sprengmittel Croscarmellose Natrium und als Schmiermittel Magnesiumstearat verwendet wird.

Weitere Träger- oder Hilfsstoffe können zugesetzt werden, wie z.B. Bindemittel, Antioxidantien, Farbstoffe, Gleitmittel, Süßungsmittel und/oder Aromastoffe.

Als Gleit- oder Schmiermittel sind bevorzugt z.B. Talk, Stärke, Magnesiumund Calciumstearat, Borsäure, Paraffin, Kakaobutter, Macrogol, Leucin oder Natriumbenzoat, ganz besonders bevorzugt ist Magnesiumstearat.

Die nachfolgenden Beispiele betreffen die Herstellung und die Zusammensetzung der erfindungsgemäßen pharmazeutischen Zubereitung:

Beispiel 1

Die folgenden Mengen werden benötigt, um beispielsweise 2 Millionen Tabletten herzustellen:

| Levothyroxin 100 µg | |
|---|---|
| Inhaltsstoff | Menge [kg] |
| | |
| Levothyroxin Natrium* | 0.210 |
| Lactose Monohydrat | 131.80 |
| Maisstärke | 50.00 |
| Gelatine | 10.00 |
| Croscarmellose Natrium | 7.00 |
| Magnesiumstearat | 1.00 |
| Wasser, gereinigt** | 56.66 |

\* Eine 5 %ige Überdosierung für Levothyroxin Natrium wurde mit eingerechnet.

\*\* Das Wasser wird durch Trocknen wieder entfernt.

Herstellung:

1. Gelatine wird in ca. 90 % des Wassers bei einer Temperatur von 80 bis 100°C gelöst.
Levothyroxin Natrium wird in ca. 10 % des Wassers bei Raumtemperatur suspendiert.
Die Suspension wird danach bei 50°C (± 5°C) zur Gelatinelösung gegeben. Die Temperatur der so erhaltenen Suspension (=Granulierflüssigkeit) ist 45 bis 50°C.

2. Lactose und Maisstärke werden in einem Wirbelschichtgranulator plaziert. Die Granulierflüssigkeit wird über das Pulver gesprüht. Die Temperatur der Granulierflüssigkeit wird während des Sprühprozesses zwischen 40 und 50°C gehalten. Während der Granulation wird die Temperatur am Inlet bei ungefähr 70° C (± 5°C), am Outlet zwischen 20 und 40°C gehalten. Der Sprühdruck liegt zwischen 3 und 5 bar.
Nach dem Sprühen wird das Granulat getrocknet, bis am Outlet eine Temperatur von ungefähr 40°C erreicht ist.

Anschließend wird das trockene Granulat nach bekannten Methoden gesiebt (1mm) (=Mischung a).

Croscarmellose Natrium und Magnesiumstearat werden entsprechend gesiebt. Anschließend werden die Komponenten zusammen mit Mischung a in einem Trommelmixer 10 Minuten miteinander vermischt.

Die pressfertige Mischung wird danach zu Tabletten verpreßt.

Beispiel 2

Zusammensetzung einer 100 mg (± 3 mg) Tablette, die 100 µg Levothyroxin Natrium enthält:

| | |
|---|---|
| Levothyroxin Natrium | 0,100 mg |
| Lactose Monohydrat | 65,90 mg |
| Maisstärke | 25,00 mg |
| Gelatine | 5,00 mg |
| Croscarmellose Natrium | 3,50 mg |
| Magnesiumstearat | 0,50 mg |
| | 100,00 mg |

Levothyroxin Natrium ist um 5 % überzudosieren.

Beispiel 3

Zusammensetzung einer 100 mg (± 3 mg) Tablette, die 100 µg Levothyroxin Natrium enthält:

| | |
|---|---|
| Levothyroxin Natrium | 0,100 mg |
| Liothyronin Natrium | 0,020 mg |
| Lactose Monohydrat | 65,88 mg |
| Maisstärke | 25,00 mg |
| Gelatine | 5,00 mg |
| Croscarmellose Natrium | 3,50 mg |
| Magnesiumstearat | 0,50 mg |
| | 100,00 mg |

Levothyroxin Natrium ist um 5 % überzudosieren.


**Patentansprüche**

1. Tablette enthaltend Levothyroxin Natrium, Gelatine und Füllstoffe und die frei von organischen Lösungsmittelrückständen ist.

2. Pharmazeutische Zubereitung nach Anspruch 1, **dadurch gekennzeichnet, dass** neben Levothyroxin Natrium auch Liothyronin Natrium enthalten ist.

3. Pharmazeutische Zubereitung nach Anspruch 1 oder 2, **dadurch gekennzeichnet, dass** 5 bis 400 µg an Levothyroxin Natrium enthalten sind.

4. Pharmazeutische Zubereitung nach einem oder mehreren der Ansprüche 1 bis 3, **dadurch gekennzeichnet, dass** mikronisiertes Levothyroxin Natrium mit einer Korngröße zwischen 5 µm und 25 µm enthalten ist.

5. Pharmazeutische Zubereitung nach einem oder mehreren der Ansprüche 1 bis 4, **dadurch gekennzeichnet, dass** Füllstoffe, ausgewählt aus der Gruppe Lactose und/oder Maisstärke und/oder mikrokristalline Cellulose enthalten sind.

6. Verfahren zur Herstellung einer pharmazeutischen Zubereitung, **dadurch gekennzeichnet, dass** man in einer Wirbelschichtgranulation Levothyroxin Natrium und gegebenenfalls Liothyronin Natrium, das/die in suspendierter Form in wässriger Gelatinelösung vorliegt/vorliegen, auf den/die Füllstoff(e) aufsprüht, anschließend ein Spreng- und Schmiermittel zumischt und die Mischung zu Tabletten verpresst.

7. Verfahren nach Anspruch 6, **dadurch gekennzeichnet, dass** als Sprengmittel Croscarmellose Natrium und als Schmiermittel Magnesiumstearat verwendet wird.

# EP 1 077 681 B1

**Claims**

1. Tablet comprising levothyroxine sodium, gelatine and fillers and which is free of organic solvent residues.

2. Pharmaceutical preparation according to Claim 1, **characterized in that**, in addition to levothyroxine sodium, liothyronine sodium is also contained.

3. Pharmaceutical preparation according to Claim 1 or 2, **characterized in that** 5 to 400 μg of levothyroxine sodium are contained.

4. Pharmaceutical preparation according to one or more of Claims 1 to 3, **characterized in that** micronized levothyroxine sodium with a particle size of between 5 μm and 25 μm is contained.

5. Pharmaceutical preparation according to one or more of Claims 1 to 4, **characterized in that** fillers selected from the group consisting of lactose and/or maize starch and/or microcrystalline cellulose are contained.

6. Process for the production of a pharmaceutical preparation, **characterized in that** levothyroxine sodium and optionally liothyronine sodium, which is/are present in suspended form in aqueous gelatine solution, are sprayed onto the filler(s) in a fluidized bed granulation, then a disintegrant and lubricant are admixed and the mixture is compressed to give tablets.

7. Process according to Claim 6, **characterized in that** the disintegrant used is croscarmellose sodium and the lubricant used is magnesium stearate.


**Revendications**

1. Comprimés contenant de la lévothyroxine sodique, de la gélatine et des matières de charge et débarrassés des résidus de solvants organiques.

2. Préparation pharmaceutique selon la revendication 1, **caractérisée en ce qu'**elle contient à côté de la lévothyroxine sodique également de la liothyronine sodique.

3. Préparation pharmaceutique selon la revendication 1 ou 2, **caractérisée en ce qu'**elle contient 5 à 400 μg de lévothyroxine sodique.

4. Préparation pharmaceutique selon l'une ou plusieurs des revendications 1 à 3, **caractérisée en ce que** la lévothyroxine sodique micronisée a une granulométrie comprise entre 5 μm et 25 μm.

5. Préparation pharmaceutique selon l'une ou plusieurs des revendications 1 à 4, **caractérisée en ce que** les matières de charge sont choisies dans le groupe constitué par le lactose et/ou l'amidon de maïs et/ou la cellulose microcristalline

6. Procédé pour la fabrication d'une préparation pharmaceutique, **caractérisé en ce que** l'on pulvérise dans un granulateur à lit fluidisé la lévothyroxine sodique et éventuellement la liothyronine sodique, se présentant sous forme de suspension dans une solution aqueuse de gélatine, sur la ou les matière(s) de charge, **en ce que** l'on ajoute ensuite un agent désintégrateur et un lubrifiant et **en ce que** l'on compresse le mélange pour former des comprimés.

7. Procédé selon la revendication 6, **caractérisé en ce que** l'on utilise comme agent désintégrateur la croscarmellose sodique et comme lubrifiant le stéarate de magnésium.